# EUROPEAN PATENT SPECIFICATION

(11) **EP 1 773 220 B1**
(45) Date of publication and mention of the grant of the patent: **03.09.2008**
(21) Application number: 05763121.0
(22) Date of filing: 28.07.2005
(51) Int. Cl.: A61B 17/66

(54) **AUTO-EXTENSIBLE DEVICE**
SELBSTERWEITERBARE VORRICHTUNG
DISPOSITIF AUTO-EXTENSIBLE

(30) Priority: 29.07.2004 GB 0417005
(43) Date of publication of application: 18.04.2007
(73) Proprietor: Finsbury (Development) Limited, Leatherhead, Surrey KT22 0BA (GB)
(72) Inventor: TAYLOR, Andrew Clive, West Sussex PO19 3QQ (GB)
(74) Representative: Smaggasgale, Gillian Helen
(86) International application number: PCT/GB2005/002959
(87) International publication number: WO 2006/010933

(56) References cited:
- EP-A- 1 240 873
- WO-A-92/22268
- US-A- 5 334 202
- US-A- 5 961 553

## Description

The present invention relates to an auto-extensible device, more particularly to an auto-extensible device which is capable of being extended in length by precisely controlled amounts, for example, precisely controlled amounts in the range of from about 40 µm to about 120 µm. Such an auto-extensible device finds utility in the medical field, for example in the field of time distractors, such as bone lengthening or bone straightening devices, as well as in the field of manned or unmanned spacecraft.

The invention thus has applicability in a number of fields, including medicine and military or civilian spacecraft.

Ilizarov discovered that new bone and soft tissue is regenerated under the effect of slow and gradual distraction which is normally effected with the aid of external fixation. This technique has been utilised in the treatment of various bone conditions. Limb length differences resulting from congenital, developmental, post-traumatic or post-surgical causes may be treated in this manner. The procedure also lends itself to the treatment of congenital deformities, post-traumatic bone deformities, non-healing fractures and bone loss from tumour, trauma or infection.

Traditionally an external bone fixator has been used which comprises a framework of metal rings connected by rods, whereby each ring is connected to the bone by a plurality of wires under tension or by pins. Titanium pins may be used to support the bone. Presently, a wide variety of designs of fixator are available and are suitable for withstanding the forces imposed by the full weight of the patient. One example is that disclosed in United States Patent Specification No. 4,615,338. Others are disclosed in U.S.S.R. Inventor's Certificates No. 848,011 and 865,284. Further designs of fixation device are to be found in United States Patent Specifications No. 5,971,984, 4,889,111, 5,062,844, 5,095,919, and 6,129,727.

In surgical limb lengthening, the bone is subjected to osteotomy so as to sever it into two or more parts before the fixator is attached to the severed parts of the bone. In the course of the operation the surgeon will attach at least one pair of pins to each of the severed parts of the bone and then join the pins externally of the patient's limb by means of a rod or rods. Generally there is at least one rod on each side of the limb. Just a few days after surgery the patient is encouraged to resume normal use of the limb in order to maintain joint flexibility and to facilitate muscle growth to match the osteogenesis.

Approximately one week after the surgery to fit the fixator, manual adjustments are commenced in order to lengthen the rods equally so as to separate the severed ends of the bone at a rate of about 1 mm per day. An increase of more than about 1 mm per day results in a slowing of the osteogenesis and an increase of less than about 1 mm per day can result in premature consolidation.

In surgical limb straightening the bone can be severed completely or partially. If the bone is completely severed, then the rod or rods on one side of the limb may be lengthened at a greater rate than the rod or rods on the other side thereof. Alternatively the bone can be partially severed according to a technique known as open wedge osteotomy, in which case the surgeon makes a cut on one side only of the bone and then a bone fixator may be needed only on that side of the bone in which the cut has been made by the surgeon.

It has further been found that osteogenesis proceeds more satisfactorily if frequent small adjustments in bone length are made by distraction rather than larger less frequent adjustments of bone length. Hence adjustments of about 0.25 mm every 6 hours are recommended. This places a burden upon the patient and carer to conform to a routine which can be very disruptive to day to day life.

It is very common for patients to experience a great deal of pain each time that the fixator is incrementally lengthened. This can make the four times daily lengthening procedure a traumatic experience both for the patient and for the patient's carer, particularly if the patient is a young child. Since the entire bone lengthening or straightening process can last from three to six months this can impose a continuing great strain not only on the patient but also on those caring for the patient. Moreover this procedure tends to lead to very high complication rates so that it is not uncommon for the complication rate to be as high as about 200% which means that each patient on average experiences at least two incidents during a course of bone lengthening or straightening treatment requiring a return to hospital, possibly for further surgery.

Another problem with external bone fixators is that there is a significant risk of infection arising at the site of each pin or wire.

It has been proposed to utilise gradual motorised distraction in which a typical procedure could involve applying a very small incremental lengthening over 1000 times per day which still achieves an average bone lengthening rate of about 1 mm per day.

In European Patent Publication No. 1 240 873 A3 there is disclosed a mechanism for powering an auto-extensible tissue distractor, such as a bone fixator, in which a movable device is caused to move in small incremental steps of a few µm each along an elongate member towards its distal end under the influence of one or more piezoelectric actuators.

United States Patent Specification No. 5,180,380 describes an orthopaedic system which includes a plurality of support members, a plurality of rods interconnecting the support members, a plurality of pins attached to the support members for passing through the bones of a patient, and an automatic drive device to control an adjustment mechanism of the rods to alter the relative positions of the support members. In this system the drive device includes at least one motor for incrementally adjusting the adjustment mechanism of at least one of the rods and a controller device for providing pulses to the motor and for storing information regarding the number of stepwise adjustments of the rod length by the motor.

United States Patent No. 5,626,579 discloses a surgically implantable cable apparatus for *in vivo* bone transport of a bone segment between a first bone segment and a second bone segment by means of a cable attached by one end to the bone segment, the other end of the cable being connected to an implantable actuator.

In United States Patent No. 5,626,581 there is described an implantable bone lengthening apparatus which includes a shape memory material-powered hydraulic pump, a shape memory material-powered ratchet mechanism, a permeable head piston mechanism and a bellows extension mechanism.

United States Patent No. 5,961,553 discloses a device for elongating long bones including an intramedullary nail with a tubular sleeve and an extension axially slidable in the sleeve with an electric motor arranged within the sleeve linked to a speed reducer driving a screw/nut assembly for moving the extension relative to the sleeve. The device also includes means for supplying power to the electric motor and automatically controlling the value and direction of the movement imparted to the sleeve by the screw/nut assembly driven by the electric motor.

A system for therapeutic treatment of a bone is described in United States Patent No. 6,022,349. This system includes a source of energy for stimulating the bone, a feedback loop for receiving response information from the bone generated by the stimulation, and an adjustment device for adjusting the energy source according to predetermined criteria.

In United States Patent No. 6,033,412 an implantable distractor is described that includes an actuator powered by intermittent electrical current flow through a shape-memory-effect actuation component.

United States Patent No. 6,383,155 B1 teaches an intramedullary nail for bone distraction that has an electric motor drive that is located in its interior and is connected with a reception antenna for feeding electrical energy via an electrical connection. The nail has an orifice which faces the reception antenna and allows the feeding of energy.

Another field in which an auto-extensible device can find acceptance is in the field of spacecraft, whether military or civilian in purpose.

There are many artificial communications satellites in orbit around the earth. These typically provide communication using radio frequencies or microwave frequencies. In addition there are telescopes on extraterrestrial satellites which require to be steered extremely accurately. Furthermore interplanetary space probes carry equipment whose orientation often needs to be controlled very precisely from the mission control station.

It is often desired, particularly with military communications satellites, to be able to adjust the position of radio frequency or microwave frequency aerials relative to the body of the satellite very precisely so that a signal beamed up from a ground station can be reflected or re-transmitted back to earth with a very tightly controlled footprint so that the reflected or re-transmitted signal can be received only by receivers positioned within that footprint. Similarly telescopes in space require a steering mechanism to enable the telescope to be pointed very precisely in a desired direction. In addition, items of equipment on interplanetary space probes often require very precise control from the mission control station.

In order to achieve the necessary precision of positioning a footprint for a re-transmitted or reflected radio frequency or microwave frequency signal, very precise control of the aerial on the extraterrestrial satellite is needed. Such aerials are typically mounted on the communications satellite by means of three support struts, at least one of which, and preferably all of which, can be altered in length under control from a control station on the ground. In order to achieve the required precision of control of the footprint of the re-transmitted or reflected radio frequency or microwave frequency signal it may be necessary to change the length of one of the support struts by at most a few µm. A similar support system can be used to support telescopes of all sorts, including radio telescopes, light telescopes, and infra-red telescopes, as well as other steerable equipment, on extraterrestrial satellites and interplanetary probes.

Since the cost per kg of putting equipment in orbit around the earth is considerable, it would be desirable to provide a lightweight auto-extensible device that can be remotely controlled and incorporated in a support strut for a radio frequency or microwave frequency aerial, a telescope, or other item of equipment in outer space environments.

There is accordingly a need for an auto-extensible device for use in medical devices such as bone lengthening or straightening devices which obviates the need for manual adjustment of the lengths of the rods providing support for the surgically severed bone, whether the bone has been totally severed or partially severed, and enables such adjustment to be achieved without significant pain being experienced by the patient.

There is a further need for a lightweight auto-extensible device for use in outer space environments whose length can be accurately controlled extremely precisely from a ground control station.

The present invention accordingly seeks to provide a novel form of auto-extensible device which can be incorporated in a bone fixator or other form of medical device, such as a bone lengthening or straightening device, whereby the length of the medical device can be imperceptibly increased in a manner such that the patient undergoing bone lengthening or straightening treatment does not experience significant pain as a result of the lengthening of the device. It further seeks to provide a lightweight auto-extensible device for extraterrestrial use whose length can be very precisely controlled from a ground control station.

According to the present invention there is provided an auto-extensible device comprising:
a first body provided with an axial bore therein;
an elongate tubular member having a proximal end, a distal end, at least one axial slot extending from the distal end, an internal bore, and an external screw thread , and the proximal end being slidably received in the axial bore of the first body;
a linear bearing guide having a portion extending through the at least one axial slot into the internal bore;
a second body slidably received on the elongate tubular member between the linear bearing guide and the first body and connected to the linear bearing guide;
a first gear ring disposed on the elongate tubular member between the first body and the second body, the first gear ring having a first internal screw thread threadedly engaged with the external screw thread on the elongate tubular member and having first peripheral gear teeth;
a first motor arranged for driving a first spur gear having first spur gear teeth in engagement with the first peripheral gear teeth;
a second gear ring disposed on the elongate tubular member between the second body and the linear bearing guide, the second gear ring having a second internal screw thread threadedly engaged with the external screw thread on the elongate tubular member and having second peripheral gear teeth;
a second motor arranged for driving a second spur gear having second spur gear teeth engaged with the peripheral second gear teeth;
electrorestrictive means mounted within the elongate tubular member and having a proximal end located with respect to the first body and having a distal end adapted to bear against the linear bearing guide; and
voltage generating means electrically connected to the electrorestrictive means for applying a voltage thereto so as to cause the electrorestrictive means to increase in length by a predetermined incremental amount.

In a cycle of operation, upon actuating the voltage generating means in a first step so as apply a predetermined voltage to the electrorestrictive means, the electrorestrictive means increases in length by an incremental amount and moves the linear bearing guide, the second body, the elongate tubular member, and the first and second gear rings by the incremental amount distally away from the first body so as to form a first gap between the first body member and the second body as well as a second gap between the first gear ring and a distal face of the first body, and then, upon actuating the first motor in a second step, the first spur gear rotates the first gear ring and drives it along the external screw thread on the elongate tubular member towards the proximal end thereof a distance substantially equal to the incremental amount until it abuts the first body, thereby to close the second gap, and then in a third step the voltage generating means ceases applying voltage to the electrorestrictive means so as to cause the electrorestrictive means to decrease in length thereby to close the first gap and to produce a third gap between the second gear ring and a distal surface of the second body, and thereafter, upon subsequently actuating the second motor in a fourth step, the second spur gear rotates the second gear ring and drives it along the external thread on the elongate tubular member towards the proximal end thereof a distance substantially equal to the incremental amount until it abuts the second body and closes the third gap (C) in readiness for a subsequent cycle of operation.

Conveniently the first motor is mounted in the first body. It is also convenient to arrange that the second motor is mounted in the second body.

Preferably the elongate tubular member is provided with a pair of diametrically opposed longitudinal slots each for passage of a corresponding portion of the linear bearing guide.

It will usually be preferred that the second body is held captive to the first body.

The retainer means may comprise a plurality of bolts or screws which are arranged to compress respective compression springs as the second body moves distally along the elongate tubular member relative to the first body.

In a preferred construction the linear bearing guide is secured to the second body by means of a plurality of screws or bolts.

In such a device the first motor can be arranged to drive the first spur gear through a first planetary gear box. Similarly the second motor can be arranged to drive the second spur gear through a second planetary gear box.

A load cell may be positioned so as to be capable of measuring the load imposed on the device.

Preferably a ball bearing joint is provided between the distal end of the electrorestrictive means and the linear bearing guide. Typically the electrorestrictive means comprises a piezoelectric actuator.

Control circuitry is preferably provided which is adapted so as to interrupt the supply of voltage to the electrorestrictive means in the event that the load across the device exceeds a predetermined value. Furthermore control circuitry may be provided which is adapted to switch off the first motor when this stalls. In this case it will usually also be preferred that control circuitry is provided which is adapted to switch off the second motor when this stalls.

In another aspect the invention also provides a bone fixator including an auto-extensible device according to the invention.

In yet another aspect of the invention there is provided a spacecraft comprising an accessory fixed thereto by means of a plurality of supports, wherein at least one of the supports comprises an auto-extensible device according to the invention. In such a spacecraft the accessory may be a radio frequency aerial or a microwave frequency aerial and the aerial may be secured to the spacecraft by means of three supports; in this case one of the supports, or each of the supports, may be provided with an auto-extensible device in accordance with the invention.

In order that the invention may be clearly understood and readily carried into effect a preferred embodiment thereof will now be described, by way of example only, with reference to the accompanying drawings, in which:
Figure 1 is a perspective view of a bone lengthening device which incorporates an auto-extensible device in accordance with the invention in a contracted condition;
Figure 2 is a left hand end view from the left of the bone lengthening device of Figure 1;
Figure 3 is a corresponding right hand end view of the bone lengthening device of Figure 1 in a contracted condition thereof;
Figures 4 to 9 are semi-diagrammatic cross sections of an auto-extensible device forming part of a bone lengthening device that is similar to that of Figures 1 to 3 showing various stages during operation of the device; and
Figure 10 is a block circuit diagram of the auto-extensible device of Figures 4 to 9.

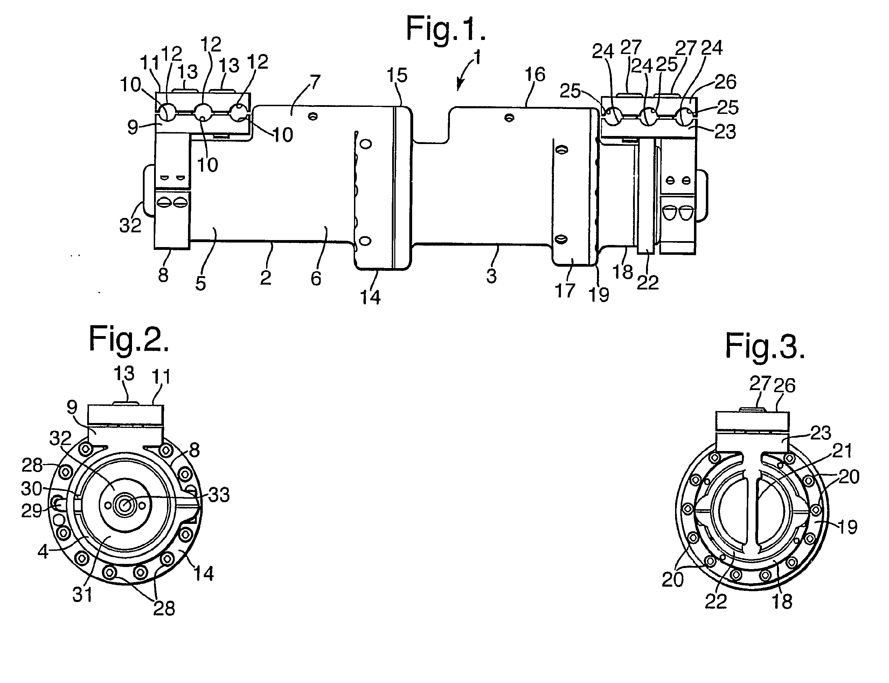

Referring to Figures 1 to 3 of the drawings, an auto-extensible bone fixator 1 comprises a first body member 2 and a second body member 3 each of which has an axial bore (not shown in Figures 1 to 3) formed therein which receives an elongate generally tubular member 4 (see Figure 2). Fixator 1 is suitable for use in bone lengthening or bone straightening procedures.

Although a rearward end portion 5 of first body member 2 is cylindrical in section, a forward end portion 6 thereof is formed with a protuberance or hump 7 which receives a first motor (also not shown in Figures 1 to 3) with a drive shaft extending parallel to the axis of the bore in first body member 2. A split collar 8 is attached at the rearward end of first body member 2. Collar 8 is formed with a flange 9 which is provided with three transverse arcuate grooves 10. A clamping plate 11 with corresponding transverse grooves 12 is held captive to flange 10 by means of screws or bolts 13 which pass through corresponding holes in clamping plate 11 and are received in corresponding bores in flange 9. Grooves 10 and 12 form apertures for reception of one or more pins or wires (not shown) which have been surgically implanted in a portion of the bone to be lengthened or straightened, e.g. a femur or a tibia, during an orthopaedic surgical operation carried out by an orthopaedic surgeon so as to sever that bone wholly or partially. By tightening the screws or bolts 13 the clamping plate 11 can be drawn towards flange 9 so that the pin or pins (or wire or wires) can be securely clamped to the fixator 1.

At its forward end first body member 2 is provided with an enlarged flange portion 14.

Second body member 3 has a corresponding flange 15 at its rearward end. As with first body member 2, a rearward end portion 15 of second body member 3 is cylindrical in section while a forward end portion has a protuberance or hump 16 which houses a second motor (also not shown in Figures 1 to 3) with a drive shaft extending substantially parallel to the axis of the cylindrical bore in second body member 3. A forward end of second body member 3 has a flange 17 by means of which it is attached to a front body 18 which carries a rear flange 19. Screws 20 (see Figure 3) pass through corresponding holes in flange 19 into respective blind bores formed in flange 17 so as to fix front body 18 to second body member 3.

A forward end of front body 18 bears on a linear bearing guide 21 which is in turn connected to a locking ring 22 that carries a flange 23, which is generally similar to flange 9. Flange 23 has three transverse arcuate grooves 24 which face corresponding arcuate transverse grooves 25 in clamping plate 26. Screws or bolts 27 hold clamping plate 26 captive to flange 24, passing through corresponding holes in clamping plate 26 into bores in flange 24. Grooves 24 and 25 together define apertures for reception of one or more pins or wires implanted into the other portion of the surgically severed bone to be lengthened or straightened. By tightening screws or bolts 27 such pins or wires can be clamped firmly to linear bearing guide 21.

Second body member 3 is held loosely captive to first body member 2 by means of bolts 28 (see Figure 2) whose threaded ends are received in corresponding blind bores in flange 15. Compression springs are positioned on the shank of each bolt 28 between its head and flange 15 so that, as illustrated, second body member 3 is urged leftward, as illustrated, towards first body member 2 in the proximal direction by these compression springs.

In Figure 2 there is visible a transverse pin 29 which extends through a longitudinal slot 30 in tubular member 4 that extends from the proximal end of tubular member 4 a part of the way only towards its distal end. Reference numeral 31 indicates an adjustable end cap, while reference numeral 32 shows an end nut which retains a load cell 33 in place.

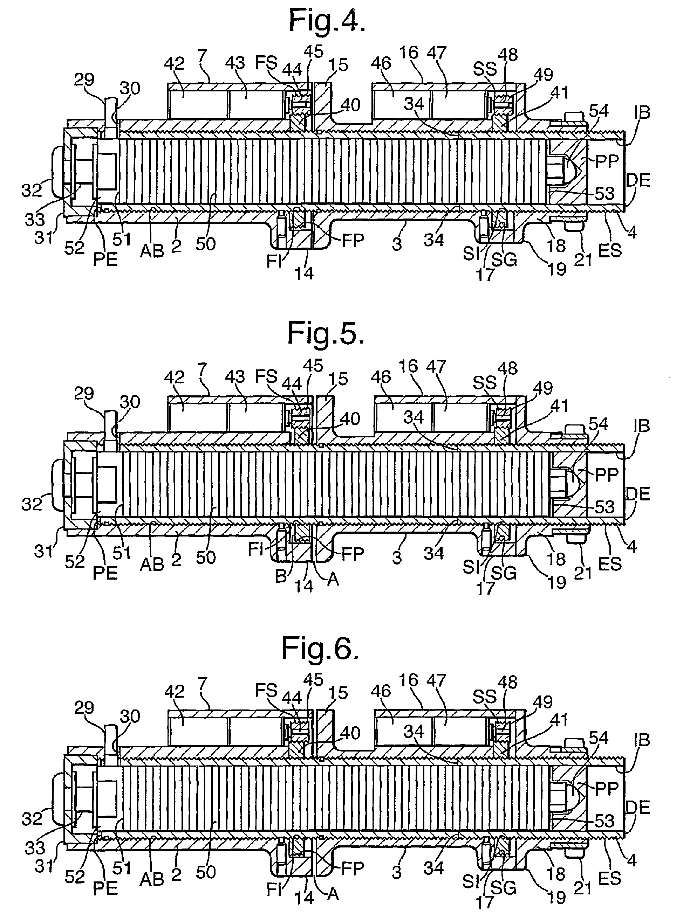

Figure 4 shows a longitudinal section through an auto-extensible device similar to that of the bone fixator, of Figures 1 to 3. One difference between the device of Figure 4 and that of the bone fixator 1 of Figures 1 to 3 is that in the device of Figure 4 pin 29 projects at an angle of 90° to the direction in which pin 29 projects in Figure 2.

First body member 2 has an axial bore AB formed therein which receive a proximal end PE of tubular member 4. Tubular member 4 has a proximal end PE, a distal end DE, a pair of axial slots (not shown) extending from the distal end DE, an internal bore IB, and an external screw thread ES. The proximal end PE of tubular member 4 is slidably received in the axial bore AB of the first body member 2.

The slots in tubular member 4 mentioned above are for passage of the ends of the linear bearing guide 21. The proximal ends of these slots are indicated by means of reference numerals 34. A portion PP of linear bearing guide 21 is thus received within the tubular member 4.

Tubular member 4 carries a first gear ring 40 and a second gear ring 41. First gear ring 40 has an internal screw thread FI, by means of which it is threadedly engaged with the external screw thread ES on tubular member 4. Similarly second gear ring 41 has an internal screw thread SI by means of which it is threadedly engaged with the external screw thread ES on tubular member 4.

A first electric motor 42 is housed within hump 7 and is arranged to drive through a first planetary gear box 43 a drive shaft 44 whose axis extends parallel to the longitudinal axis of tubular member 4. Drive shaft 44 carries a first spur gear 45 whose gear teeth FS engage with peripheral gear teeth FP of first gear ring 40. For reasons which will appear below, the teeth of first spur gear 45 and first gear ring 40 can slide relative to one another in the axial direction of tubular member 4.

Hump 16 houses a second electric motor 46. This drives through a second planetary gear box 47 a second drive shaft 48, whose axis is substantially aligned with that of first drive shaft 44. This second drive shaft 48 carries a second spur gear 49 whose gear teeth SS engage with peripheral gear teeth on second gear ring 41. For a reason which will appear below second spur gear 49 and second gear ring 41 can slide relative to one another in the axial direction of tubular member 4.

Because of the interaction between tubular member 4 and pin 29, and between tubular member 4 and linear bearing guide 21, tubular member 4 is prevented from turning about its axis. Instead it can only move longitudinally relative to the first body member 2 and the second body member 3.

A piezoelectric actuator 50 is slidably mounted coaxially within the first body member 2 and also within tubular member 4. Its proximal end 51 is received within first body member 2 and bears against a proximal guide piece 52 which also receives one end of load cell 33, while its distal end 53 bears through a ball bearing 54 against linear bearing guide 21. Ball bearing 54 thus acts as a spherical bearing so as to ensure that piezoelectric actuator 50 does not experience any bending.

Piezoelectric actuator 50 comprises a stack of piezoelectric crystals. A typical material for the piezoelectric crystals is lead zirconate titanate. The individual piezoelectric crystals are each sandwiched between a respective pair of electrodes to which an electric potential can be applied. Moreover each piezoelectric crystal is insulated from its neighbours. Upon application of an electric potential of from about 100 volts to about 1000 volts across each of the crystals of actuator 50, the entire stack extends by a small amount, e.g. up to about 120 µm, in a direction parallel to the longitudinal axis of first body member 2. In such a stroke of the piezoelectric actuator 50 it exerts a force of up to 3000 Newtons.

The mode of operation of the auto-extensible mechanism of Figure 4 will now be further described with reference also to Figures 5 to 9. As can be seen in Figure 4, in the "start" position, the left hand side (as depicted) of first gear ring 40 and also that of second gear ring 41 are abutted against the adjacent part of the first body member 2 and the second body member 3 respectively. Upon a suitable voltage being applied across the piezoelectric crystals of actuator 50 at a controlled rate of increase of voltage, it extends in length and bearing guide 21, front body 18, and second body member 3 are caused to move distally with respect to first body 2 through a distance corresponding to the stroke of piezoelectric actuator 50 of up to about 120 µm to the position shown in Figure 5. (In each of Figures 5 to 9 the distances through which the various items move and the gaps between different items have in each case been greatly exaggerated, for the sake of clarity of understanding). In the course of this movement of second body member 3 the compression springs on the shafts of bolts 28 become compressed. In addition, second body member 3 also causes second gear ring 41 to move distally a corresponding distance and hence tubular member 4 is also caused to move distally relative to first body member 2 through a similar distance of up to about 120 µm. Tubular member 4 also causes first gear ring 40 to move distally through a similar distance relative to first body member 2 and to cause a gap A to appear between the distal end of first body member 2 and the proximal end of second body member 3. As this movement occurs, the teeth SS of first spur gear 45 also slide axially relative to the teeth FP of first gear ring 40 and another gap B opens up between first gear ring 40 and the adjacent part of first body member 2. Both of gaps A and B can be seen in Figure 5.

As a result of this extension of the piezoelectric actuator 50, split collar 8 and ring 22 (see Figure 1) are forced apart by a corresponding amount of up to about 120 µm and so the respective bone portions connected to split collar 8 and to ring 22 are also forced apart by the same distance.

The rate of movement can be controlled by control of the rate at which the voltage is applied across the individual piezoelectric crystals of piezoelectric actuator 50. The load cell 33 allows the load being transmitted between the split collar and ring 22 to be monitored so as to ensure that no undue amount of force is applied to the bone being treated. The control circuitry is arranged so that, if the load cell 33 detects, during increase in the voltage applied to the piezoelectric actuator 50, that the force applied by the piezoelectric actuator 50 is about to exceed a predetermined value, then the increase in voltage is immediately halted until the load 33 indicates that it is safe to continue to increase the voltage being applied.

While maintaining the voltage across the individual crystals of the piezoelectric actuator 50 corresponding to the desired increase in length of piezoelectric actuator 50, first motor 42 is then actuated so as to rotate first spur gear 45 and hence to rotate first gear ring 40 about tubular member 4 and cause it to move in the proximal direction along tubular member 4 and to close gap B until first gear ring 40 again abuts against the adjacent part of first body member 2, as shown in Figure 6. In the course of this movement the teeth FS of first spur gear 45 slide axially with respect to the teeth FP of first gear ring 40. As will further explained below suitable control circuitry can be provided to detect when motor 42 stalls, whereupon motor 40 is immediately switched off.

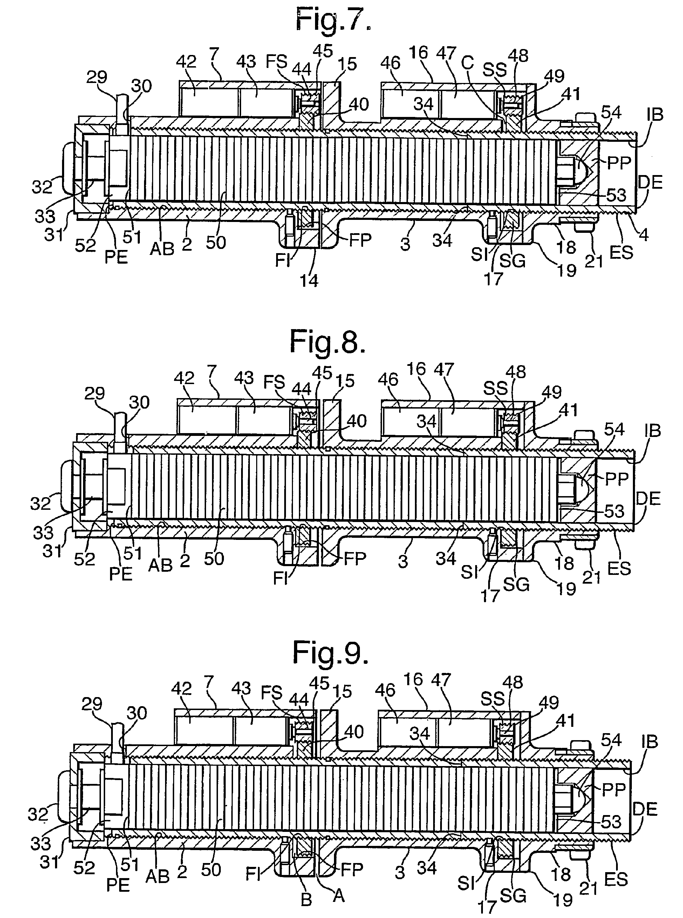

The piezoelectric actuator 50 is then de-activated by switching off the voltage applied across its individual crystals so that it returns to its original length and its distal end 53 disengages from linear bearing guide 21. This creates a gap C between gear ring 41 and the adjacent proximal end portion second body member 3, as shown in Figure 7.

In the final step of the operating cycle, second motor 46 is actuated so as to rotate second spur gear 49 and to cause gear ring 41 to move in the proximal direction so as to close gap C. During this movement the teeth SS of second spur gear 49 slide axially relative to the external teeth SG of second gear ring 41. As second gear ring 41 abuts the distal end of second body member 3, second motor 46 stalls and the control circuitry switches off second motor 46. At the end of this step the position is as indicated in Figure 8. This is essentially the same as the position illustrated in Figure 4 except that tubular member 4 has been moved distally with respect to the first body member 2 by a distance corresponding to the stroke of the piezoelectric actuator 50.

This cycle of operations can then be repeated.

Figure 9 shows the position at the end of the first step of the next cycle, the piezoelectric actuator 50 having been caused to extend by a distance of up to about 120 µm so as to cause tubular member 4 to move distally with respect to the first body member 2 by a further distance corresponding to the stroke of the piezoelectric actuator 50. The other steps are then conducted as described with reference to Figures 6 to 8.

The sequence of four steps described in relation to Figures 5 to 8 provides an "inchworm" technique by means of which the illustrated auto-extensible device can undergo movement of its elongate tubular member 4 in the distal direction with respect to first body member 2 in incremental steps. Such a technique can thus provide substantially continuous lengthening of bone fixator 1 throughout the patient's waking hours (and possibly also during his or her sleeping hours), without causing significant pain levels to the patient.

If desired, a low amplitude oscillatory signal, for example, having a frequency of from about 5 Hz to about 2 kHz can be superimposed on the voltage potentials applied to the crystals of the piezoelectric actuator 50, with a view to providing enhancement to the process of osteogenesis.

Preferably the extension caused by the application of the selected voltage potential to piezoelectric actuator 50 and the number of cycles per day for which this procedure is repeated are selected so as to give a rate of movement of the elongate tubular member 4 relative to first body member 2 of about 1 mm per day.

If desired, an oscillatory signal can be applied at some point during the stroke so long as the amplitude of the high frequency signal is less than the extension already caused by the voltage potential at the time that the oscillatory signal is applied. Conveniently the oscillatory signal is applied after the full extension has been achieved. However, it can be applied before the full extension has been achieved, if desired. Such an oscillatory signal can be, for example, a frequency, typically a sine wave frequency, of about 5 Hz to about 1 kHz, having an amplitude of not more than about 10 µm and is preferably applied after the peak extension caused by the voltage potential has been achieved, for example, after the extension of piezoelectric actuator 50 has reached about 40 µm out of its maximum permissible extension of about 120 µm, but before the first motor 42 is operated. At all events, in order not to cause damage to the piezoelectric actuator 50, the amplitude of any oscillatory signal must not exceed the extension caused by the d.c. voltage potential on which the oscillatory signal is superimposed.

It is of course not necessary always to apply the maximum possible safe operating voltage potential to the piezoelectric actuator 50. Thus, for example, even if the maximum permissible extension achievable by piezoelectric actuator 50 is about 120 µm, the designer of the bone fixator, or the orthopaedic surgeon supervising its use, may decide that the tubular member 4 shall move in each stroke only, for example, about 40 µm. This has the advantage that lower peak voltage potentials can be used, thus reducing the risk of the external insulation of the bone fixator 1 breaking down and allowing the patient to suffer electric shocks. For example, the surgeon may decide that application of 25 cycles per day each of about 40 µm will provide the desired distraction rate of approximately 1 mm per day, even though the maximum safe permissible extension of the piezoelectric actuator may be about 120 µm.

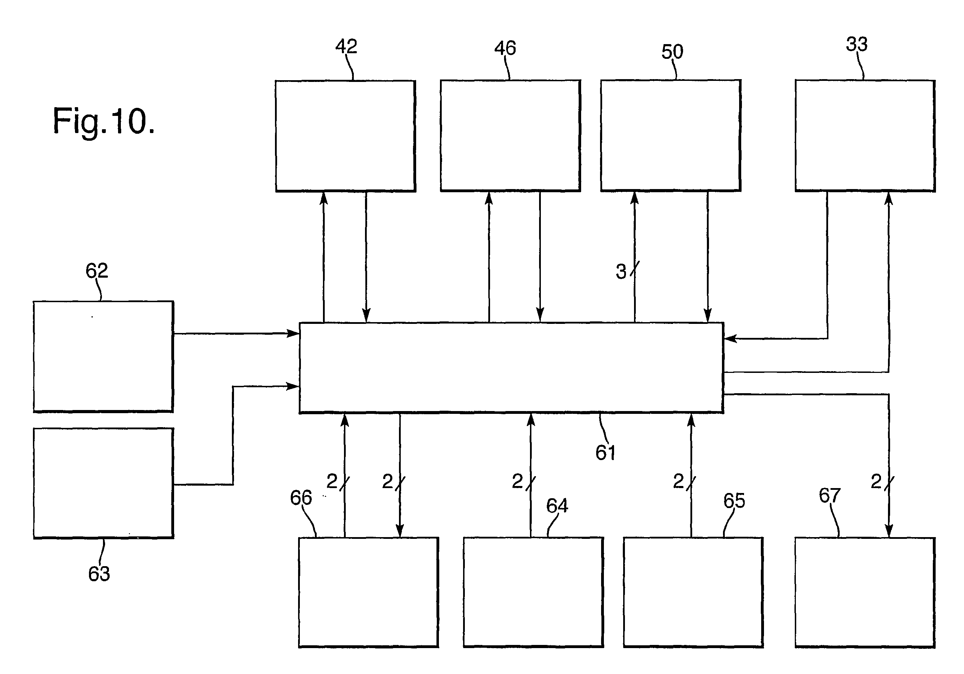

Figure 10 is an electronic block diagram of the bone fixator 1. The heart of the circuit is a microprocessor 61 which receives inputs from the bobbin load cell 33, which consists of a strain gauge bridge. It also receives inputs from two distance sensors 62, 63, feedback from the motors 18 and 29 as well as from the actuator 41, a timer clock 64, and a processor clock 65. Distance sensors 62, 63 comprise respective counters using LEDs on the gear teeth on first and second spur gears 45, 49 to determine the global lengthening, as well as respective capacitative distance sensors. The combination of techniques provided by the two distance sensors 62, 63 permit accurate compliance determination of the loaded bone site. The timer clock 64 is a programmable low frequency oscillator used to wake the microprocessor 61 up from sleep in order to save power. An RS232 communications port 66 is provided to enable the doctor, surgeon or other medical staff overseeing treatment to program set-up parameters and read data stored in data logging memory 67 which has been gathered post clinical procedure.

The microprocessor 61 is an integrated circuit connected to a microprocesor clock. Typically this is a 4 MHz ceramic resonator while the timer clock 64 is a 32 kHz watch crystal with its associated clock integrated circuit which incorporates the oscillator circuit and a frequency divider to provide a 1 Hz output.

At the hospital or clinic, or at the surgeon's consulting rooms, the parameters required for controlling the rate of extension of fixator 1 can be input into the microprocessor 61 from an input device, such as a personal computer. Such parameters may include the rate of ramping the voltage applied to the piezoelectric actuator 50.

Although the bone fixator 1 will often be used as an external device fitted to pins or wires which extend through the patient's skin to the respective bone portion to which they are surgically attached, the bone fixator 1 can be implanted into a patient's body, either internally of the patient's bone or else externally thereof. In this event, the electronic control circuitry will include a wireless interface or similar interface so that the surgeon can interrogate the memory and program the circuitry to effect changes in the more of action of the bone fixator 1. Moreover a battery can also be incorporated in the bone fixator 1 to provide power for motors 42 and 46 and for providing by means of suitable circuitry the necessary voltage for operation of the piezoelectric actuator 50.

Tissue distractors provided with an auto-extensible device in accordance with the invention may also find other uses in surgery. For example, in cases in which the shape of the spine requires to be corrected, tissue distractors maybe fitted one on each side of the patient's spinal column, each being connected to at least two vertebrae. By then extending one distractor at a greater rate than the other it can be attempted to remedy malformations and misalignments of the spinal column. Other usages which can be envisaged for tissue distractors in accordance with the invention include cosmetic surgery, for example for changing the shape of a patient's nose, cheek bone, or lower jaw.

Further uses of a tissue distractor in accordance with the invention will be readily apparent to those skilled in the art.

It is also clear to those skilled in the art that the auto-extensible mechanism illustrated in Figures 4 to 9 has many other applications, for example, in manned or unmanned spacecraft in which it can be used as one of a plurality of support devices for a radio antenna or similar device so that by adjusting the length of the auto-extensible device the precise orientation of a radio antenna relative to the spacecraft can be adjusted. For example, a radio dish antenna can be mounted on a military or civilian spacecraft on three support devices, each incorporating an auto-extensible device of the type illustrated in Figures 4 to 9. By appropriately lengthening a selected one of the three support devices the orientation of the radio dish antenna or other item of equipment can be adjusted very precisely. Such an adjustment can be used, for example, to move the footprint of a radio frequency signal beamed to the spacecraft from a ground station and reflected from the dish or other radio aerial mounted on the spacecraft over the surface of the earth so as to limit reception of the radio frequency signal only to persons located within that footprint.

In some circumstances, for example, in extraterrestrial environments, it may be desirable to enable the illustrated auto-extensible device 1 to shorten in length from an already extended condition. This can be achieved by providing at least one spring connection between the first body member 2 and the linear bearing guide 21 which acts in a proximal direction with respect to the first body member 2. In this case the piezo-electric actuator 50, during extension of the auto-extensible device 1 acts against the spring connection or connections with a force greater than the return force provided by the spring connection or connections. During shortening of the auto-extensible device 1 the piezo-electric actuator 50 is not used. Appropriate adjustments to the control circuitry and to the cycle of operation are made to facilitate the auto-contraction of the device 1, in this case.

It is also envisaged that in an extraterrestrial environment the auto-extensible device 1 can be used to damp out any vibrations that may be set up in a support strut that includes the device 1 by utilising the piezoelectric actuator 50 to damp out the vibrations without attempting to change the relative positions of the first body member 2 and the linear bearing guide 21.

## Claims

1. An auto-extensible device comprising:
a first body (2) provided with an axial bore (AB) therein;
an elongate tubular member (4) having a proximal end (PE), a distal end (DE), at least one axial slot (34) extending from the distal end (DE), an internal bore (IB), and an external screw thread (ES), and the proximal end (PE) being slidably received in the axial bore (AB) of the first body (2);
a linear bearing guide (21) having a portion (PP) extending through the at least one axial slot (34) into the internal bore (IB);
a second body (3) slidably received on the elongate tubular member (4) between the linear bearing guide (21) and the first body (2) and connected to the linear bearing guide (21);
a first gear ring (40) disposed on the elongate tubular member (4) between the first body (2) and the second body (3), the first gear ring (40) having a first internal screw thread (FI) threadedly engaged with the external screw thread (ES) on the elongate tubular member (4) and having first peripheral gear teeth (FP);
a first motor (42) arranged for driving a first spur gear (45) having first spur gear teeth (FS) in engagement with the first peripheral gear teeth (FP);
a second gear ring (41) disposed on the elongate tubular member (4) between the second body (3) and the linear bearing guide (21), the second gear ring (41) having a second internal screw thread (SI) threadedly engaged with the external screw thread (ES) on the elongate tubular member (4) and having second peripheral gear teeth (SG);
a second motor (46) arranged for driving a second spur gear (49) having second spur gear teeth (SS) engaged with the peripheral second gear teeth (SG);
electrorestrictive means (50) mounted within the elongate tubular member (4) and having a proximal end (51) located with respect to the first body (2) and having a distal end (53) adapted to bear against the linear bearing guide (21); and
voltage generating means electrically connected to the electrorestrictive means (50) for applying a voltage thereto so as to cause the electrorestrictive means (50) to increase in length by a predetermined incremental amount;
whereby, in a cycle of operation, upon actuating the voltage generating means in a first step so as apply a predetermined voltage to the electrorestrictive means (50), the electrorestrictive means (50) increases in length by an incremental amount and moves the linear bearing guide (21), the second body (3), the elongate tubular member (4), and the first and second gear rings (40, 41) by the incremental amount distally away from the first body (2) so as to form a first gap (A) between the first body member (2) and the second body (3) as well as a second gap (B) between the first gear ring (40) and a distal face of the first body (2), and then, upon actuating the first motor (42) in a second step, the first spur gear (45) rotates the first gear ring (40) and drives it along the external screw thread (ET) on the elongate tubular member (4) towards the proximal end (PE) thereof a distance substantially equal to the incremental amount until it abuts the first body (2), thereby to close the second gap (B), and then in a third step the voltage generating means ceases applying voltage to the electrorestrictive means (50) so as to cause the electrorestrictive means (50) to decrease in length thereby to close the first gap (A) and to produce a third gap (C) between the second gear ring (41) and a distal surface of the second body (3), and thereafter, upon subsequently actuating the second motor (46) in a fourth step, the second spur gear (49) rotates the second gear ring (41) and drives it along the external thread (ET) on the elongate tubular member (4) towards the proximal end (PE) thereof a distance substantially equal to the incremental amount until it abuts the second body (2) and closes the third gap (C) in readiness for a subsequent cycle of operation.

2. An auto-extensible device according to Claim 1, wherein the first motor (42) is mounted in the first body (2).

3. An auto-extensible device according to Claim 1 or Claim 2, wherein the second motor (46) mounted in the second body (3).

4. An auto-extensible device according to any one of Claims 1 to 3, wherein the elongate tubular member (4) is provided with a pair of diametrically opposed longitudinal slots each for passage of a corresponding portion of the linear bearing guide (21).

5. An auto-extensible device according to any one of Claims 1 to 4, wherein the second body (3) held captive to the first body (2).

6. An auto-extensible device according to Claim 5, wherein the retainer means (25) comprise a plurality of bolts or screws (28) which are arranged to compress respective compression springs as the second body (3) moves distally along the elongate tubular member (4) relative to the first body (2).

7. An auto-extensible device according to any one of Claims 1 to 6, wherein the linear bearing guide (21) is secured to the second body (3) by means of a plurality of screws or bolts (20).

8. An auto-extensible device according to any one of Claims 1 to 7, wherein the first motor (42) is arranged to drive the first spur gear (45) through a first planetary gear box (43).

9. An auto-extensible device according to any one of Claims 1 to 8, wherein the second motor (46) is arranged to drive the second spur gear (49) through a second planetary gear box (47).

10. An auto-extensible device according to any one of Claims 1 to 9, wherein a load cell (33) is positioned so as to be capable of measuring the load imposed on the device.

11. An auto-extensible device according to any one of Claims 1 to 10, wherein a ball bearing joint (54) is provided between the distal end (53) of the electrorestrictive means (50) and the linear bearing guide (21).

12. An auto-extensible device according to any one of Claims 1 to 11, wherein the electrorestrictive means (50) comprises a piezoelectric actuator.

13. An auto-extensible device according to any one of Claims 1 to 12, wherein control circuitry is provided which is adapted so as to interrupt the supply of voltage to the electrorestrictive means (50) in the event that the load across the device exceeds a predetermined value.

14. An auto-extensible device according to any one of Claims 1 to 13, wherein control circuitry is provided which is adapted to switch off the first motor (42) when this stalls.

15. An auto-extensible device according to any one of Claims 1 to 14, wherein control circuitry is provided which is adapted to switch off the second motor (46) when this stalls.

16. A bone fixator including an auto-extensible device according to any one of Claims 1 to 15.

17. A spacecraft comprising an accessory fixed thereto by means of a plurality of supports, wherein at least one of the supports comprises an auto-extensible device according to any one of Claims 1 to 15.

18. A spacecraft according to Claim 17, wherein the accessory is a radio frequency aerial or a microwave frequency aerial and wherein the aerial is secured to the spacecraft by means of three supports.

## Patentansprüche

1. Selbst verlängerbare Vorrichtung mit
einem ersten Körper (2) mit einer in ihm vorgesehenen Axialbohrung (AB),
einem länglichen rohrförmigen Element (4) mit einem proximalen Ende (PE), einem distalen Ende (DE), wenigstens einem von dem distalen Ende (DE) ausgehenden axialen Schlitz (34), einer inneren Bohrung (IB) und einem äußeren Schraubgewinde (ES), wobei das proximale Ende (PE) in der Axialbohrung (AB) des ersten Körpers (2) verschiebbar aufgenommen ist,
einer geradlinigen Lagerführung (21) mit einem sich durch den wenigstens einen axialen Schlitz (34) in die innere Bohrung (IB) erstreckenden Teil (PP),
einem zweiten Körper (3), der auf dem länglichen rohrförmigen Element (4) zwischen der geradlinigen Lagerführung (21) und dem ersten Körper (2) verschiebbar aufgenommen und mit der geradlinigen Lagerführung (21) verbunden ist,
einem auf dem länglichen rohrförmigen Element (4) zwischen dem ersten Körper (2) und dem zweiten Körper (3) angeordneten ersten Getriebering (40) mit einem ersten inneren Schraubgewinde (FI), das mit dem äußeren Schraubgewinde (ES) auf dem länglichen, rohrförmigen Element (4) verschraubt ist und erste, auf dem Umfang befindliche Getriebezähne (FP) hat,
einem ersten Motor (42) zum Antrieb eines ersten Stirnrades (45) mit ersten Stirnradzähnen (FS) in Eingriff mit den ersten, auf dem Umfang befindlichen Getriebezähnen (FP),
einem auf dem länglichen, rohrförmigen Element (4) zwischen dem zweiten Körper (3) und der geradlinigen Lagerführung (21) angeordneten zweiten Getriebering (41) mit einem zweiten inneren Schraubgewinde (SI), das mit dem äußeren Schraubgewinde (ES) auf dem länglichen, rohrförmigen Element (4) verschraubt, ist und zweite, auf dem umfang befindliche Getriebezähne (SG) hat,
einem zweiten Motor (46) zum Antrieb eines zweiten Stirnrades (49) mit zweiten Stirnradzähnen (SS) in Eingriff mit den zweiten, auf dem Umfang befindlichen Getriebezähnen (SG),
einem in dem länglichen rohrförmigen Element (4) angebrachten elektrorestriktiven Mittel (50) mit einem in Bezug auf den ersten Körper (2) angeordneten proximalen Ende (51) und einem zum Tragen gegen die gradlinige Lagerführung (21) eingerichteten distalen Ende (53) und
einem Spannungsgenerator, der an das elektrorestriktive Mittel (50) elektrisch angeschlossen ist, um eine Spannung an dieses anzulegen und das elektrorestriktive Mittel (50) so zu veranlassen, sich um einen vorbestimmten Zusatzbetrag zu verlängern,
wobei in einem Betrriebszyklus in einer ersten Stufe bei Einschaltung des Spannungsgenerators zur Anlegung einer vorbestimmten Spannung an das elektrorestriktive Mittel (50) dieses in der Lange um einen Zuwachsbetrag zunimmt und die geradlinige Lagerführung (21), den zweiten Körper (3), das längliche, rohrförmige Element (4) und den ersten und zweiten Getriebering (40, 41) von dem ersten Körper (2) um einen Zuwachsbetrag distal wegbewegt, um so zwischen dem ersten Körperelement (2) und dem zweiten Körper (3) einen ersten Spalt (A) und zwischen dem ersten Getriebering (40) und der distalen Seite des ersten Körpers (2) einen zweiten Spalt (B) zu bilden, und dann in einer zweiten Stufe bei Einschaltung des ersten Motors (42) das erste Stirnrad (45) den ersten Getriebering (40) dreht, und auf dem länglichen rohrförmigen Element (4) längs des äußeren Schraubgewindes (ES) zu dem proximalen Ende (PE) des Elements über eine Strecke antreibt, die im wesentlichen bleich dem Zuwachsbetrag ist, bis der Ring auf den ersten Körper (2) trifft, um so den zweiten Spalt (B) zu schließen, und dann in einer dritten Stufe der Spannungsgenerator die an dem elektrorestriktiven Mittel (50) anliegende Spannung abstellt, um so das elektrorestriktive Mittel (50) zu einer Verkürzung zum veranlassen und **dadurch** den ersten Spalt (A) zu schließen und zwischen dem zweiten Getriebering (41) und der distalen Seite des zweiten Körpers (3) einen dritten Spalt (C) zu bilden, und danach in einer vierten Stufe durch anschließende Einschaltung des zweiten Motors (46) das zweite Stirnrad (49) den zweiten Getriebering (41) dreht und ihn auf dem länglichen rohrförmigen Element (4) längs des äußeren Schraubgewindes (ES) zur seinem proximalen Ende (PE) hin eine dem Zuwachsbetrag im wesentlichen gleiche Strecke treibt, bis er auf den zweiten Körper (2) trifft und zur Bereitschaft für einen folgenden Betriebszyklus den dritteln Spalt (C) schließt.

2. Selbst verlängerbare Vorrichtung nach Anspruch 1, bei der der erste Motor (42) in dem ersten Körper (2) angebracht ist.

3. Selbst verlängerbare Vorrichtung nach Anspruch 1 oder Anspruch 2, bei der der zweite Motor (46) in dem zweiten Körper (3) angebracht ist.

4. Selbst verlängerbare Vorrichtung nach einem der Anspruche 1 bis 3, bei der das längliche rohrförmige Element (4) mit einem Paar sich diametral gegenüberliegender Längsschlitze jeweils für den Durchgang eines entsprechenden Teils der geradlinigen Lagerführung (21) versehen ist.

5. Selbst verlängerbare Vorrichtung nach einem der Ansprüche 1 bis 4, bei der der zweite Körper (3) an dem ersten Körper (2) festgehalten wird.

6. Selbst verlängerbare Vorrichtung nach Anspruch 5, bei der das Haltemittel (25) mehrere Bolzen oder Schrauben (28) umfaßt, die für sie Kompression von Druckfedern eingerichtet sind, wenn sich der zweite Körper (3) relativ zur dem ersten Körper (2) distal längs des länglichen rohrförmigen Elements (4) bewegt.

7. Selbst verlängerbare Vorrichtung nach einem der Ansprüche 1 bis 6, bei der die geradlinige Lagerführung (21) mittels mehrerer Schraubern oder Bolzen (20) an dem zweiten Körper (3) befestigt, ist.

8. Selbst verlängerbare Vorrichtung nach einem der Ansprüche 1 bis 7, bei der der erste Motor (42) zum Antrieb des ersten Stirnrades (45) durch einen ersten Planetengetriebekasten (43) eingerichtet ist.

9. Selbst verlängerbare Vorrichtung nach einem der Anspruche 1 bis 8, bei der der zweite Motor (46) zum Antrieb des zweiten Stirnrades (49) durch einen zweiten Planetengetriebekasten (47) eingerichtet ist.

10. Selbst verlängerbare Vorrichtung nach einem der Ansprüche 1 bis 9, bei der eine Lastzelle (33) so angeordnet ist, dass sie die auf die Vorrichtung einwirkende Last messen kann.

11. Selbst verlängerbare Vorrichtung nach einem der Ansprüche 1 bis 10, bei der zwischen dem distalen Ende (53) des elektrorestriktiven Mittels (50) und der geradlinigen Lagerführung (21) eine Kugellagervebindung (54) vorgesehen ist.

12. Selbst verlängerbare Vorrichtung nach einem der Anspruche 1 bis 11, bei der das elektrorestriktive Mittel (50) ein piezoelektrisches Betätigungselement umfaßt.

13. Selbst verlängerbare Vorrichtung nach einem der Anspruche 1 bis 12. bei der eine Steuerschaltung vorgesehen ist, die so eingerichtet ist, dass sie die Spannungsversorgung für das elektrorestriktive Mittel (50) in dem Falle unterbricht, dass die belastung an der Vorrichtung einen vorbestimmten Wert übersteigt.

14. Selbst verlängerbare Vorrichtung nach einem der Anspruche 1 bis 13, bei der eine Steuerschaltung vorgesehen, ist, die zur Abschaltung des ersten Motors (42) bei dessen Blockierung eingerichtet ist.

15. Selbst verlängerbare Vorrichtung nach einem der Ansprüche 1, bis 14, bei der eine Steuerschaltung vorgesehen ist, die zur Abschaltung des zweiten Motors (46) bei dessen Blockierung eingerichtet ist.

16. Knochenfixator unter Einschluß eines selbst verlängerbaren Geräts nach einem der Ansprüche 1 bis 15.

17. Raumfahrzeug mit einem Zusatzgerät, das durch mehrere Träger an ihm befestigt ist, wobei wenigstens einer der Träger eine selbst verlängerbare Vorrichtung nach einem der Ansprüche 1 bis 15 aufweist.

18. Raumfahrzeug nach Anspruch 17, bei dem das Zusatzgerät eine Hochfrequenzantenne oder eine Mikrowellenfrequenzantenne ist und bei dem die Antenne durch drei Träger an dem Raumfahrzeug befestigt, ist.

## Revendications

1. Dispositif auto-extensible, comprenant :
un premier corps (2) muni d'un alésage axial (AB) dans celui-ci ;
un élément tubulaire allongé (4) ayant une extrémité proximale (PE), une extrémité distale (DE), au moins une fente axiale (34) s'étendant à partir de l'extrémité distale (DE), un alésage interne (IB), et un filetage externe (ES), l'extrémité proximale (PE) étant reçue en coulissement dans l'alésage axial (AB) du premier corps (2) ;
un guide de roulement linéaire (21) ayant une partie (PP) qui s'étend à travers la au moins une fente axiale (34) dans l'alésage interne (IB) ;
un second corps (3) reçu en coulissement sur l'élément tubulaire allongé (4) entre le guide de roulement linéaire (21) et le premier corps (2) et relié au guide de roulement linéaire (21) ;
un premier anneau d'engrenage (40) disposé sur l'élément tubulaire allongé (4) entre le premier corps (2) et le second corps (3), le premier anneau d'engrenage (40) ayant un premier filetage interne (FI) engagé de manière filetée avec le filetage externe (ES) sur l'élément tubulaire allongé (4), et ayant une première denture périphérique (FP) ;
un premier moteur (42) agencé de manière à entraîner un premier engrenage droit (45) ayant une première denture d'engrenage droit (FS) en prise avec la première denture périphérique (FP) ;
un second anneau d'engrenage (41) disposé sur l'élément tubulaire allongé (4) entre le second corps (3) et le guide de roulement linéaire (21), le second anneau d'engrenage (41) ayant un second filetage interne (SI) engagé de manière filetée avec le filetage externe (ES) sur l'élément tubulaire allongé (4) et ayant une seconde denture périphérique (SG) ;
un second moteur (46) agencé de manière à entraîner un second engrenage droit (49) ayant une seconde denture d'engrenage droit (SS) en prise avec la seconde denture périphérique (SG) ;
des moyens électrostrictifs (50) montés à l'intérieur de l'élément tubulaire allongé (4) et ayant une extrémité proximale (51) positionnée par rapport au premier corps (2) et ayant une extrémité distale (53) adaptée pour reposer contre le guide de roulement linéaire (21) ; et
des moyens de génération de tension électriquement raccordés aux moyens électrostrictifs (50) pour apporter une tension à ceux-ci de sorte à provoquer une augmentation incrémentielle prédéterminée de la longueur des moyens électrostrictifs (50) ;
moyennant quoi, dans un cycle opérationnel, suite à l'actionnement, à une première étape, des moyens de génération de tension pour appliquer une tension prédéterminée aux moyens électrostrictifs (50), la longueur des moyens électrostrictifs (50) augmente d'une quantité incrémentielle et déplace le guide de roulement linéaire (21), le second corps (3), l'élément tubulaire allongé (4), ainsi que le premier et le second anneau d'engrenage (40,41) d'une quantité incrémentielle à l'écart du premier corps (2) de manière distale, de sorte à former un premier espace (A) entre le premier élément de corps (2) et le second corps (3) ainsi qu'un deuxième espace (B) entre le premier anneau d'engrenage (40) et une face distale du premier corps (2), puis, suite à l'actionnement du premier moteur (42), à une deuxième étape, le premier engrenage droit (45) entraîne en rotation le premier anneau d'engrenage (40) et l'entraîne le long du filetage externe (ET) sur l'élément tubulaire allongé (4) vers l'extrémité proximale (PE) de celui-ci sur une distance sensiblement égale à la quantité incrémentielle, jusqu'à ce qu'il bute contre le premier corps (2), fermant ainsi le deuxième espace (B), et ensuite, à une troisième étape, les moyens de génération de tension cessent d'appliquer la tension aux moyens électrostrictifs (50) de sorte à provoquer une diminution de la longueur des moyens électrostrictifs (50) pour fermer le premier espace (A) et produire un troisième espace (C) entre le second anneau d'engrenage (41) et une surface distale du second corps (3) et, suite à cela, dès l'actionnement subséquent du second moteur (46) à une quatrième étape, le second engrenage droit (49) entraîne en rotation le second anneau d'engrenage (41) et l'entraîne le long du filetage externe (ET) sur l'élément tubulaire allongé (4) vers l'extrémité proximale (PE) de celui-ci, à une distance sensiblement égale à la quantité incrémentielle, jusqu'à ce qu'il bute contre le second corps (2) et ferme le troisième espace (C) en étant prêt pour un cycle opérationnel subséquent.

2. Dispositif auto-extensible selon la revendication 1, dans lequel le premier moteur (42) est monté dans le premier corps (2).

3. Dispositif auto-extensible selon la revendication 1 ou 2, dans lequel le second moteur (46) est monté dans le second corps (3).

4. Dispositif auto-extensible selon l'une quelconque des revendications 1 à 3, dans lequel l'élément tubulaire allongé (4) est muni d'une paire de fentes longitudinales diamétralement opposées dont chacune est destinée au passage d'une partie correspondante du guide de roulement linéaire (21).

5. Dispositif auto-extensible selon l'une quelconque des revendications 1 à 4, dans lequel le second corps (3) est retenu attaché au premier corps (2).

6. Dispositif auto-extensible selon la revendication 5, dans lequel les moyens de retenue (25) comprennent une pluralité de boulons ou de vis (28) qui sont agencés de sorte à comprimer des ressorts de compression respectifs à mesure que le second corps (3) se déplace de manière distale le long de l'élément tubulaire allongé (4) par rapport au premier corps (2).

7. Dispositif auto-extensible selon l'une quelconque des revendications 1 à 6, dans lequel le guide de roulement linéaire (21) est fixé au second corps (3) au moyen d'une pluralité de vis ou de boulons (20).

8. Dispositif auto-extensible selon l'une quelconque des revendications 1 à 7, dans lequel le premier moteur (42) est agencé de manière à entraîner le premier engrenage droit (45) à travers une première boîte d'engrenages planétaires (43).

9. Dispositif auto-extensible selon l'une quelconque des revendications 1 à 8, dans lequel le second moteur (46) est agencé de manière à entraîner le second engrenage droit (49) à travers une seconde boîte d'engrenages planétaires (47).

10. Dispositif auto-extensible selon l'une quelconque des revendications 1 à 9, dans lequel une cellule de charge (33) est positionnée de manière à être apte à mesurer la charge imposée sur le dispositif.

11. Dispositif auto-extensible selon l'une quelconque des revendications 1 à 10, dans lequel un joint à billes (54) est positionné entre l'extrémité distale (53) des moyens électrostrictifs (50) et le guide de roulement à billes (21).

12. Dispositif auto-extensible selon l'une quelconque des revendications 1 à 11, dans lequel les moyens électrostrictifs (50) comprennent un actionneur piézoélectrique.

13. Dispositif auto-extensible selon l'une quelconque des revendications 1 à 12, dans lequel une circuiterie de commande est fournie qui est adaptée pour interrompre l'alimentation en tension vers les moyens électrostrictifs (50) dans le cas où la charge à travers le dispositif dépasse une valeur prédéterminée.

14. Dispositif auto-extensible selon l'une quelconque des revendications 1 à 13, dans lequel une circuiterie de commande est fournie qui est adaptée pour éteindre le premier moteur (42) quand celui-ci cale.

15. Dispositif auto-extensible selon l'une quelconque des revendications 1 à 14, dans lequel une circuiterie de commande est fournie qui est adaptée pour éteindre le second moteur (46) quand celui-ci cale.

16. Elément de fixation osseuse comprenant un dispositif auto-extensible selon l'une quelconque des revendications 1 à 15.

17. Engin spatial comprenant un accessoire fixé à celui-ci au moyen d'une pluralité de supports, dans lequel au moins un des supports comprend un dispositif auto-extensible selon l'une quelconque des revendications 1 à 15.

18. Engin spatial selon la revendication 17, dans lequel l'accessoire est une antenne de fréquences radioélectriques ou une antenne d'hyperfréquences et dans lequel l'antenne est fixée à l'engin spatial au moyen de trois supports.
